# EUROPEAN PATENT APPLICATION

(11) **EP 2 250 912 A1**
(43) Date of publication of application: **17.11.2010**
(21) Application number: 10161580.5
(22) Date of filing: 30.04.2010
(51) Int. Cl.: A23L 1/30, A23L 1/303, A61K 35/74

(54) **A health-enhancing preparation administrable in the form of drops, and a method for preparing the same**

(30) Priority: 06.05.2009 FI 20095512
(71) Applicant: Oriola, OY, 02200 Espoo (FI)
(72) Inventor: Niemi, Jukka, FI-00630, Helsinki (FI); Kiuru, Jarkko, FI-03100, Nummela (FI); Tammersalo-Karstén, Ina, FI-02120, Espoo (FI); Sundholm, Susanna, FI-00680, Helsinki (FI); Aaltonen, Jari, FI-00410, Helsinki (FI)
(74) Representative: Saijonmaa, Olli-Pekka

(57) **Abstract**

The invention relates to a non-aqueous health-enhancing preparation to be administered in the form of drops, and a method for preparing the same. The product substantially consists of edible oil, such as sunflower oil, and lactic acid bacteria *Lactobacillus rhamnosus* GG and vitamin D suspended in it. In addition to these, the composition of the product may comprise a chemically non-reacting anti-caking agent, such as silicon dioxide and/or an antioxidant. The product is prepared by first mixing vitamin D into edible oil and then suspending the lactic acid bacteria. The product is intended particularly for expectant or breastfeeding mothers or infants, to strengthen the child's immune response, to inhibit diarrhoea and to promote the development of the skeletal system.

## Description

The invention relates to a non-aqueous health-enhancing preparation to be administered in the form of drops, containing lactic acid bacteria suspended in edible oil. The invention relates to a method for preparing such a product, as well as a product that can be prepared by the method.

Lactic acid bacteria (*Lactobacillus*) are one of the most vital microbial groups present in the intestines of a healthy person. Lactic acid bacteria are probiotic, that is, they have a beneficial effect on human health by countering the growth of harmful, pathogenic bacteria in the intestines. By maintaining a healthy intestinal system they promote digestion and the absorption of minerals contained in food. In small children, the lactic acid bacteria promote the development of an immune response and thereby prevent diarrhoea. Other manifested beneficial effects of the lactic acid bacteria include, among other things, lowering the cholesterol level and preventing the development of cancer of the large intestine.

Because of the advantageous effects of the lactic acid bacteria, functional foods or preparations containing them have been developed, in which the bacteria can be administered in the form of food supplements to people, especially children or expectant or breastfeeding mothers. The food supplements are represented by drops available on the market, containing the lactic acid bacterium *Lactobacillus reuteri* suspended in edible oil. The product is reported to counter allergy, diarrhoea and the adverse effects of an antibiotic regime in infants. In adults, they have been reported to reduce intestinal infections and colds. Except for drops to be administered separately, *L. reuteri* has also been used as a supplement in dairy products, such as yogurt, cheese or ice cream, baby food and juices.

Patent publications discussing *L. reuteri* include, for example, EP 357 673, in which the bacterium is described as a probiotic; EP 698 347, in which the bacterium is added into food; EP 814 822, in which the bacterium is used for preventing diarrhoea; WO 2004/034808, in which the bacterium is added into a medicine or a food product to improve immunity; and WO 2005/117532, in which the bacterium in combination with vegetable oil is described to inhibit inflammations in an infant, effective via breast milk.

Subject of extensive research, another well-known lactic acid bacterium with similar beneficial health effects is *Lactobacillus rhamnosus GG.* This strain of bacteria was described in US patent 4,839,281. In WO 2004/069178, it is presented together with *L. reuteri* as an anti-inflammatory agent suspendable in vegetable oil. *L. rhamnosus GG* also improves colonization resistance against harmful bacteria and prevents allergies by modifying lactic proteins to a less allergizing form. Thus, the administration of *L. rhamnosus GG* to an expectant or breastfeeding mother or a child has been found advantageous, and capsules, powdery products as well as functional foods, such as juices and dairy products, containing it are available on the market.

Other health effects of *L. rhamnosus GG* that have come up in studies or that are expected on the basis of them include, for example, the reduction of rheumatoid arthritis, arthritis, pneumonitis, and respiratory infections in children. Furthermore, studies have shown that *L. rhamnosus GG* reduces the risk of cancer of the urinary bland (found in animal experiments) and cancer of the large intestine.

It is an aim of the invention to provide a non-aqueous health-enhancing product in the form of drops containing lactic acid bacteria with an expanded spectrum of the above-described beneficial effects and, simultaneously, with an intensified action based on their parallel feature. The method according to the invention is **characterized in that** vitamin D is admixed with edible oil, and lactic acid bacteria of the strain *Lactobacillus rhamnosus GG* are suspended in the resulting oil-based mixture.

The preparation according to the invention, which can be prepared by the above-mentioned method, is **characterized in that** the preparation substantially consists of edible oil, lactic acid bacteria of the strain *Lactobacillus rhamnosus GG,* vitamin D, and, optionally, a chemically non-reacting anti-caking agent and/or an antioxidant.

The starting point for the invention is the fact that besides drops based on edible oil and containing lactic acid bacteria, similar oil-based drops of vitamin D have been on the market for a long time already, their primary target group being the same as that for the lactic acid bacteria drops, namely small children. Furthermore, drops of bacteria as well as drops of vitamin D are also recommended for adults, and both lactic acid bacteria and vitamin D have been added in food products which are not intended for any special group but the aggregate consumers. The present invention thus implements the availability of lactic acid bacteria and vitamin D in a single product that is easy to administer. In the case of oil-based drops which the user himself/herself can add to food or drink to be taken, the intake of lactic acid bacteria and vitamin D can be easily adjusted to meet exactly the needs of the user in question.

Fat-soluble vitamin D which is produced in a human body by the effect of light and is also obtained from food is active in the metabolism of calcium and phosphorus by promoting their absorption from nutrition and their binding in the skeletal system. Lack of vitamin D causes rickets in children and bone changes in adults, such as osteomalacia and osteoporosis. Because of the development of the skeletal system of the child, an expectant mother and an infant typically have a deficiency of vitamin D, which should be countered by administering vitamin drops or supplementing food with the vitamin, particularly in Finland and in other northern latitudes. However, an insufficient intake of vitamin D is a problem not only in children but in the whole population. In addition to vitamin drops, there are breast milk substitutes and gruels supplemented with vitamin D and intended for children. Vitamin D is also added in edible fats and some milk products and juices which are intended for the whole population.

Recent studies have shown that vitamin D affects not only bones but also health in a much wider sense. Research has shown that vitamin D may prevent cardiovascular diseases, multiple sclerosis, rheumatoid arthritis, diabetes, neoplasms, such as prostatic cancer, pneumonitis, and susceptibility to infections. These effects are partly congruent with the manifested effects of *Lactobacillus rhamnosus GG.* Furthermore, it is obvious that the lactic acid bacteria which maintain intestinal health have a beneficial effect on intestinal functions, such as the absorption of calcium available in food, where vitamin D plays an essential role as well. Consequently, *L. rhamnosus GG* and vitamin D included in the drop product according to the invention are assumed to have a synergetic effect, even if this has not been studied in detail so far.

Irrespective of what has been said above, the preparation according to the invention is particularly beneficial for mothers and infants who may obtain an essential lactic acid bacteria supplement and an essential vitamin D supplement in a suitable ratio from one and the same drop product.

About prior art, it should be mentioned that WO 02/060510 describes well a consistent viscose carrier which is supplemented with lactic acid bacteria, such as *L. rhamnosus GG,* and optionally with vitamins, which are not defined in more detail. The product is intended to be administered primarily to animals. Similarly related to the invention is WO 2005/115341, in which a product in a particle form may include *L. rhamnosus GG* as a bacterium and, for example, vitamins as an optional supplement. Neither publication discusses a drop product or mentions vitamin D or presents its effects on health. Document BE 1017714 describes a product intended for pregnant women, combining the whole spectrum of different vitamins (A, B, C, E, and E) and micronutrients (including Fe, Mg, Mn, Zn, Cu, J, Se) needed, *L. rhamnosus,* as well as carriers and other additives in a preparation primarily in the form of a tablet.

In the method according to the invention, in the first step, vitamin D is admixed with edible oil. An optional antioxidant may be admixed in the same step. An optional anti-caking agent, which should be chemically non-reacting with the other ingredients of the preparation and which is preferably silicon dioxide, is then added into the mixture. Finally, *L. rhamnosus GG* lactic acid bacteria are dispersed into the mixture.

Vitamin D included in the product according to the invention is particularly advantageously vitamin D3 (cholecalciferol). Alternatively, it can be replaced in part or in whole with vitamin D2 (ergocalciferol).

The suitable content of vitamin D in the preparation is 1 to 200 µg, particularly advantageously 10 to 25 µg of vitamin D in one gram of oil.

The suitable concentration of *Lactobacillus rhamnosus GG* in the product is 10⁷ to 10¹¹ CFU, advantageously 10⁹ to 10¹⁰ CFU in one gram of oil (CFU = colony forming units).

The oil substrate for the preparation is preferably vegetable oil, such as sunflower, soybean, rapeseed, olive, palm, or coconut oil.

Besides edible oil, lactic acid bacteria and vitamin D, the preparation may contain other necessary technical agents.

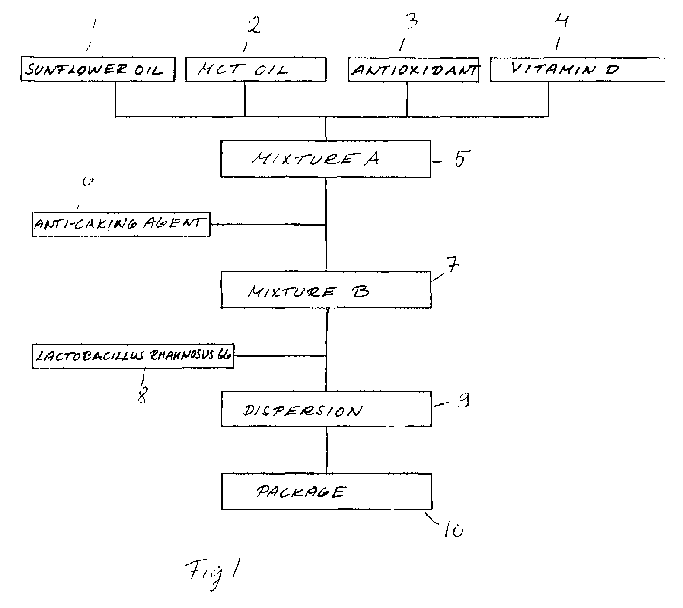

In the following, the invention will be illustrated by describing the preparation of an example product. A diagram of the method of preparation is shown in the appended Figure 1.

### Preparation of the product

The product to be prepared is a health-enhancing preparation administrable in the form of drops, containing lactic acid bacteria *Lactobacillus rhamnosus GG* suspended in sunflower oil and also containing vitamin D3. Sunflower oil 1, medium chain length triglyceride oil (MCT) 2, antioxidant, such as alpha tocopherol, gamma tocopherol, delta tocopherol or tocopherol extract 3, and vitamin D3 (cholecalciferol) 4 are mixed in a mixer (in-line mixer or paddle mixer) 5. The dosage of the vitamin 4 may be 1 to 200 µg per administered dose (0.4 g). An anti-caking agent, such as, for example, amorphous silicon dioxide 6, is added into the mixture. Mixing is performed with a mixer 7, which may be of a type similar to that in the preceding step 5. This is followed by admixing lactic acid bacteria *Lactobacillus rhamnosus GG* 8, which is of the human probiotic strain ATCC 5310 and is powdered and lyophilized or in another suitable form. The dosage may be 10⁷ to 10¹¹ CFU, particularly advantageously 5 × 10⁹ CFU per administered dose (0.4 g). If necessary, the bacterium may be mechanically ground to a finer form and dewatered before admixing. Dispersion is performed with a mixer 9, which may be of a type similar to that in the earlier steps 5 and 7. Finally, the running suspension 10 obtained as the final product is packed in consumer packages.

The following table shows the composition of a particularly advantageous example product as well as the range of content of each component.

| | example | range |
|---|---|---|
| administered dose | 0.4 g | 0.1 to 40 g |
| vegetable oil | 0.28 g | 0.01 to 35 g |
| MCT oil | 0.09 g | 0.01 to 35 g |
| antioxidant | 0.01 g | 0.001 to 10 g |
| silicon dioxide etc. | 0.01 g | 0.001 to 10 g |
| vitamin D | 0.01 g | 0.001 to 0.2 g |
| LGG | 5 × 10⁹ CFU | 10⁷ to 10¹¹ CFU |

It will be obvious for a person skilled in the art that the different embodiments of the invention are not limited to those presented as examples above, but they may vary within the scope of the appended claims.

## Claims

1. A method for preparing a non-aqueous health-enhancing preparation administrable as drops, comprising a strain of lactobacillus suspended in edible oil, **characterized in that** the edible oil is mixed with vitamin D, and lactobacillus rhamnosus GG is suspended in the oil-based mixture.

2. A method according to claim 1, **characterized in that** a chemically non-reacting anti-caking agent is added to the mixure before lactobacillus rhamnosus GG is suspended therein.

3. A method according to claim 1 or 2, **characterized in that** an antioxidant is brought into the mixture before lactobacillus rhamnosus GG is suspended therein.

4. A method according to any one of the preceding claims, **characterized in that** the preparation substantially consists of an edible oil, lactobacillus rhamnosus GG, vitamin D and, optionally, a chemically non-reacting anti-caking agent and/or an antioxidant.

5. A non-aqueous health-enhancing preparation obtainable by a method according to any one of the preceding claims, the preparation being administrable as drops and comprising a strain of lactobacillus suspended in edible oil, **characterized in that** the preparation substantially consists of an edible oil, lactobacillus rhamnosus GG, vitamin D and, optionally, a chemically non-reacting anti-caking agent and/or an antioxidant.

6. A health-enhancing preparation according to claim 5, **characterized in that** the preparation comprises vitamin D3.

7. A health-enhancing preparation according to claim 5 or 6, **characterized in that** the preparation comprises 1-200 µg, preferably 10-25 µg of vitamin D per 1 g of edible oil.

8. A health-enhancing preparation according to any one of claims 5-7, **characterized in that** the concentration of lactobacillus rhamnosus GG in the preparation is 10⁷-10¹¹, preferably 10⁹-10¹⁰ cfu (colony forming units) per 1 g of edible oil.

9. A health-enhancing preparation according to any one of claims 5-8, **characterized in that** the edible oil is a vegetable oil, such as soya, rape or sunflower oil.

10. A health-enhancing preparation according to any one of claims 5-9, **characterized in that** the anti-caking agent is silica (SiO₂).
